# EUROPÄISCHE PATENTSCHRIFT

(11) **EP 1 307 490 B1**
(45) Veröffentlichungstag und Bekanntmachung des Hinweises auf die Patenterteilung: **18.04.2007**
(21) Anmeldenummer: 01967009.0
(22) Anmeldetag: 10.08.2001
(51) Int. Cl.: C07K 19/00, C07K 16/46, C07K 17/14, G01N 30/02, C07K 16/28, C07K 16/08, C07K 16/44, C07K 16/40

(54) **FV-KONSTRUKTE MIT BEEINFLUSSBARER AFFINITAT ZU EINER ZU BINDENDEN SUBSTANZ**
Fv- CONSTRUCTS WITH AN INFLUENCEABLE AFFINITY FOR A SUBSTANCE THAT IS TO BE LINKED
STRUCTURE FV PRESENTANT UNE AFFINITE INFLUENCABLE AVEC UNE SUBSTANCE A LIER

(30) Priorität: 11.08.2000 DE 10040046; 05.09.2000 DE 10044002
(43) Veröffentlichungstag der Anmeldung: 07.05.2003
(73) Patentinhaber: Ruprecht-Karls-Universität Heidelberg, 69117 Heidelberg (DE)
(72) Erfinder: Dübel, Stefan, 69221 Dossenheim (DE)
(74) Vertreter: Schüssler, Andrea
(86) Internationale Anmeldenummer: PCT/DE2001/003098
(87) Internationale Veröffentlichungsnummer: WO 2002/014371

(56) Entgegenhaltungen:
- GRIEP REMKO A ET AL: "pSKAP/S: An expression vector for the production of single-chain Fv alkaline phosphatase fusion proteins." PROTEIN EXPRESSION AND PURIFICATION, Bd. 16, Nr. 1, Juni 1999 (1999-06), Seiten 63-69, XP002186589 ISSN: 1046-5928
- HAYASHI N ET AL: "A single expression system for the display, purification and conjugation of single-chain antibodies" GENE, ELSEVIER BIOMEDICAL PRESS. AMSTERDAM, NL, Bd. 160, Nr. 1, 4. Juli 1995 (1995-07-04), Seiten 129-130, XP004042191 ISSN: 0378-1119
- CASEY J L ET AL: "Purification of bacterially expressed single chain Fv antibodies for clinical applications using metal chelate chromatography" JOURNAL OF IMMUNOLOGICAL METHODS, ELSEVIER SCIENCE PUBLISHERS B.V.,AMSTERDAM, NL, Bd. 179, Nr. 1, 13. Februar 1995 (1995-02-13), Seiten 105-116, XP004021105 ISSN: 0022-1759
- HELFRICH W ET AL: "A rapid and versatile method for harnessing scFv antibody fragments with various biological effector functions" JOURNAL OF IMMUNOLOGICAL METHODS, ELSEVIER SCIENCE PUBLISHERS B.V.,AMSTERDAM, NL, Bd. 237, Nr. 1-2, April 2000 (2000-04), Seiten 131-145, XP004192501 ISSN: 0022-1759
- KIPRIYANOV SERGEY M ET AL: "Affinity purification of tagged recombinant proteins using immobilized single chain Fv fragments." ANALYTICAL BIOCHEMISTRY, Bd. 244, Nr. 1, 1997, Seiten 189-191, XP002186590 ISSN: 0003-2697
- KURUCZ ISTVAN ET AL: "A bacterially expressed single-chain Fv construct from the 2B4 T-cell receptor." PROCEEDINGS OF THE NATIONAL ACADEMY OF SCIENCES OF THE UNITED STATES, Bd. 90, Nr. 9, 1993, Seiten 3830-3834, XP002186591 1993 ISSN: 0027-8424
- GOEL^A A ET AL: "Relative position of the hexahistidine tag effects binding properties of a tumor-associated single-chain Fv construct" BBA - GENERAL SUBJECTS, ELSEVIER SCIENCE PUBLISHERS, NL, Bd. 1523, Nr. 1, 1. September 2000 (2000-09-01), Seiten 13-20, XP004276666 ISSN: 0304-4165

## Beschreibung

Die vorliegende Erfindung befaßt sich mit dem Gebiet der Fᵥ-Konstrukte, deren Affinität zu einer zu bindenden Substanz beeinflußt werden kann, beispielsweise kann die Bindung zwischen dem Fᵥ-Konstrukt und der Substanz wieder gelöst werden, ohne daß beide eine wesentliche Beeinträchtigung erfahren. Ferner betrifft die Erfindung ein Verfahren zur Herstellung eines solchen Fᵥ-Konstruktes sowie dessen Verwendung zur Anreicherung und Reinigung von Substanzen.

Fᵥ-Konstrukte sind in der Regel Konstrukte aus den variablen Regionen eines Antikörpers (nachstehend mit Fᵥ-Antikörper-Konstrukte bezeichnet) oder eines T Zell-Rezeptors (nachstehend mit Fᵥ-T Zell-Rezeptor-Konstrukte bezeichnet), wobei die variablen Regionen als Bindungsstelle für eine zu bindende Substanz, z.B. ein Antigen oder einen Liganden, dienen. Eine Bindungsstelle eines Fᵥ-Antikörper-Konstruktes kann dabei aus einer V_{H}- und einer V_{L}-Region, aus zwei V_{H}-Regionen, z.B. wenn die Bindungsstelle aus einem Kamel-Antikörper stammt, oder aus zwei V_{L}-Regionen gebildet sein, wenn die Bindungsstelle aus einem Bence-Jones Protein stammt. Eine Bindungsstelle eines Fᵥ-T Zell-Rezeptor-Konstruktes kann aus einer α- und einer β-Region gebildet sein. Fᵥ-Konstrukte liegen häufig einzelkettig vor, d.h. die variablen Regionen sind über Peptidlinker miteinander verbunden. Solche Fᵥ-Konstrukte werden als scFᵥ-Konstrukte bezeichnet. Sie können mono-, bi- oder oligospezifisch sein, d.h. sie weisen Bindungsstellen für eine,zwei oder mehrere verschiedene Substanzen auf.

Fᵥ-Antikörper-Konstrukte werden häufig zur Anreicherung und Reinigung von Substanzen, z.B. Proteinen, Rezeptoren, Viren etc., verwendet. Dies erfolgt z.B. in Affinitätschromatographie-Verfahren, bei denen die Fᵥ-Antikörper-Konstrukte an eine Matrix, z.B. eine Chromatographiesäule, gebunden werden, über die die Substanzen enthaltenden Lösungen gegeben werden, wodurch die Substanzen an die entsprechenden Fᵥ-Antikörper-Konserukte binden können. Durch Elution werden dann die angereicherten Substanzen erhalten. Dieser Schritt erfolgt zumeist unter harten Bedingungen, z.B. hochmolaren Salzlösungen, Lösungen mit stark saurem oder basischem pH oder mittels chaotroper/denaturierender Agentien, insbesondere dann, wenn die Affinität der Fᵥ-Antikörper-Konstrukte zu den Substanzen hoch ist. Damit werden aber sowohl die Fᵥ-Antikörper-Konstrukte als auch die Substanzen wesentlich beeinträchtigt, was oft dazu führt, daß die Fᵥ-Antikörper-Konstrukte und somit die Chromatographiesäule nicht mehr verwendbar sind und die Substanzen ihre Aktivität verlieren.

Der vorliegenden Erfindung liegt somit die Aufgabe zugrunde, ein Mittel bereitzustellen, mit dem an Fᵥ-Antikörper-Konstrukte gebundene Substanzen wieder abgelöst werden können, ohne daß die vorstehenden Nachteile für die Fᵥ-Antikörper-Konstrukte bzw. die Substanzen eintreten.

Erfindungsgemäß wird dies durch die Gegenstände in den Patentansprüchen erreicht.

Die vorliegende Erfindung beruht auf den Erkenntnissen des Anmelders, daß die Affinität eines Fᵥ-Konstruktes zu einer zu bindenden Substanz beeinflußt werden kann, wodurch z.B. die gebundene Substanz wieder abgelöst werden kann, in dem die die Bindungsstelle an den Fᵥ-Konstrukten bildenden variablen Regionen gegeneinander verschoben werden. Ferner hat er erkannt, daß hierfür keine vorstehenden Elutionsbedingungen notwendig sind und daß auch die Fᵥ-Konstrukte und die Substanzen keine wesentliche Beeinträchtigung, z.B. in ihrer Wiederverwendbarkeit bzw. Aktivität, erfahren. Vielmehr hat er gefunden, daß die gegenseitige Verschiebung der variablen Regionen z.B. durch die Bindung von Effektormolekülen, wie Ionen oder Antikörper, an entsprechende Bindungsstellen auf mit den variablen Regionen verbundenen Peptiden erfolgen kann. Bei der vorliegenden Erfindung werden die Bindungseigenschaften der Fv-Konstrukte nachträglich nach abgeschlossener Produktion und Faltung geändert und nicht während des Herstellungsprozesses. Dies bedeutet, daß es sich um eine allosterische Modulation an bereits fertig produzierten und gefalteten Antikörperfragmenten handelt. Es wird auf die nachstehenden Beispiele verwiesen.

Erfindunsgemäß werden diese Erkenntnisse des Anmelders zur Bereitstellung eines Fᵥ-Konstruktes mit beeinflußbarer Affinität zu einer zu bindenden Substanz genutzt, wobei das Fᵥ-Konstrukt mit den variablen Regionen verbundene, Bindungsstellen für Effektormoleküle enthaltende Peptide aufweist, wobei die variablen Regionen über die Peptide miteinander verbunden sind.

Der verwendete Ausdruck "Fᵥ-Konstrukt" betrifft ein Fᵥ-Konstrukt, dessen variable Regionen Peptide mit Bindungsstellen für Effektormoleküle aufweisen. Die Bindung der variablen Regionen, die über die Peptide miteinander verbunden sind, kann z.B. über eine Disulfidbrücke oder eine Peptidbindung erfolgen. Vorzugsweise sind die Peptide über eine Peptidbindung miteinander verbunden, wodurch das Fᵥ-Konstrukt als ein einzelkettiges (sc)Fᵥ-Konstrukt vorliegt. Das Fᵥ-Konstrukt kann mono-, bi- oder oligospezifisch sein, d.h. es kann Bindungsstellen für eine oder mehrere Substanzen, wie Proteine, Rezeptoren, Antikörper, Liganden, Viren, synthetische Verbindungen, etc., aufweisen. Vorzugsweise ist ein Fᵥ-Konstrukt ein Fᵥ-Antikörper-Konstrukt oder ein Fᵥ-T Zell-Rezeptor-Konstrukt, wobei es günstig sein kann, wenn diese Fᵥ-Konstrukte auch konstante Regionen aufweisen. Besonders bevorzugt weist ein Fᵥ-Konstrukt Bindungsstellen für ein Adeno-assoziiertes Virus (AAV) bzw. ein Capsid davon auf. Desweiteren kann das Fᵥ-Konstrukt ein Polymer, z.B. ein Dimer oder Tetramer, eines scFᵥ-Konstruktes sein, wobei das Polymer durch Aneinanderlagerung von mehreren scFᵥ-Konstrukten gebildet ist.

Der verwendete Ausdruck "beeinflußbare Affinität des Fᵥ-Konstruktes zu einer zu bindenden Substanz" betrifft sowohl die Verstärkung als auch die Reduzierung einer solchen Affinität, ohne daß das Fᵥ-Konstrukt und die Substanz wesentlich, z.B. in ihrer Wiederverwendbarkeit bzw. Aktivität, beeinträchtigt werden. Insbesondere ist unter der Beeinflußung der Affinität des Fᵥ-Konstruktes zu der zu bindenden Substanz die Wiederablösung der gebundenen Substanz von dem Fᵥ-Konstrukt zu verstehen, was eine große Bedeutung z.B. für die Anreicherung und Reinigung einer Substanz mittels einer Affinitätschromatographie hat.

Der verwendete Ausdruck "Peptide mit Bindungsstellen für Effektormoleküle" betrifft (Poly)peptide jeglicher Art und Abstammung, die mit variablen Regionen verbunden sein und Bindungsstellen für Effektormoleküle aufweisen können. Die Peptide können in der Natur vorkommende, in die gegebenenfalls solche Bindungsstellen eingefügt werden, oder synthetische Peptide sein. Die Peptide der einzelnen variablen Regionen sind miteinander verbunden, wobei dies z.B. über eine Disulfidbrücke oder eine Peptidbindung erfolgen kann. Als Effektormoleküle sind jegliche Effektormoleküle zu nennen, die durch Bindung an ihre in den Peptiden vorliegenden Bindungsstellen, wobei von letzteren ein oder mehrere pro Peptid vorliegen können, eine gegenseitige Verschiebung der variablen Regionen eines Fᵥ-Konstruktes bewirken. Vorzugsweise sind die Effektormoleküle Antikörper oder Ionen, z.B. bi- oder trivalente Ionen, insbesondere Metallionen, wobei ganz besonders Nickel-, Kupfer- und Zinkionen bevorzugt sind. Als Bindungsstellen für Antikörper sind deren Epitope auf entsprechenden Antigenen zu nennen, während für Metallionen, insbesondere Zinkfinger und His-Repeats, als Bindungsstellen zu erwähnen sind. Vorzugsweise weist die für Nickelionen als Effektormolekül geeignete Bindungsstelle die Aminosäuresequenz HPHHHHE auf. Besonders bevorzugt ist es, wenn die Effektormoleküle sich wieder leicht von den Bindungsstellen auf dem Peptidlinker lösen lassen, was z.B. bei Metallionen wie, Nickel-, Kupfer-, und Zinkionen, über Chelatbildner, wie EDTA oder EGTA, erfolgen kann. Ferner wird auf die nachstehenden Beispiele verwiesen. Casey et al, Journal of Immunological Methods, Vol.179 (1995) 105-116 beschreibt ein scFv mit einem Hexahistidin als Bindungsstelle am Carboxyterminalen Ende des variablen Domänes .

In bevorzugter Ausführungsform liegt ein vorstehendes Fᵥ-Konstrukt in Verbindung mit einer Matrix vor. Diese kann jeglicher fester Träger sein, an dem das Fᵥ-Konstrukt über übliche Verfahren, z.B. über ungepaarte Cysteinreste oder das Avidin/Streptavidin-Verfahren, gebunden werden kann. Vorzugsweise ist der Träger eine Chromatographiesäule, wodurch das Fᵥ-Konstrukt besonders für die Affinitätschromatographie geeignet ist.

Ein erfindungsgemäßes Fᵥ-Konstrukt kann durch verschiedene Verfahren hergestellt werden. Soll z.B. ein erfindungsgemäßes scFᵥ-Konstrukt hergestellt werden, kann ein Verfahren günstig sein, bei dem eine DNA, die für variable Regionen kodiert, wobei die Regionen über Bindungsstellen für Effektormoleküle aufweisende Peptide miteinander verbunden sind, in einem Expressionsvektor inseriert und über diesen in Zellen exprimiert wird. Aus den Zellen bzw. dem Zellüberstand kann dann das erfindungsgemäße Fᵥ-Konstrukt isoliert und gereinigt werden. Günstig ist es, wenn das Fᵥ-Konstrukt über einen Phagen-Display-Expressionsvektor, z.B. pSEX81 an der Oberfläche von Phagen präsentiert wird, wodurch seine Identifikation erleichtert wird. Es wird auf die nachstehenden Beispiele verwiesen. Hinsichtlich der Ausdrücke "Fᵥ-Konstrukt" und "Peptide mit Bindungsstellen für Effektormoleküle" wird auf vorstehende Ausführungen verwiesen. Ergänzend wird auf Maniatis, T. et al., Molecular Cloning, A Labatory Mannual, Cold Spring Harbor Labaratory 1982, verwiesen.

Ein weiterer Gegenstand der vorliegenden Erfindung ist ein Kit umfassend:
(a) ein erfindungsgemäßes Fᵥ-Konstrukt,
(b) eine erfindungsgemäße DNA, insbesondere einen erfindungsgemäßen Expressionsvektor, sowie
(c) übliche Hilfsstoffe, wie Puffer, Lösungsmittel und Kontrollen.

Von den einzelnen Komponenten können ein oder mehrere Vertreter vorliegen.

Die vorliegende Erfindung stellt ein Fᵥ-Konstrukt bereit, dessen Affinität zu einer zu bindenden Substanz beeinflußt werden kann, wodurch z.B. das Wieder-Ablösen der gebundenen Substanz erreicht werden kann. Kennzeichnend hierfür ist, daß die Beeinflußung der Affinität derart schonend ist, daß das Fᵥ-Konstrukt und die Substanz nicht wesentlich beeinträchtigt werden, so daß sie sowohl wiederverwendbar sind als auch aktiv bleiben. Damit liefert die vorliegende Erfindung ein Mittel, das sich bestens für die Anreicherung und Reinigung von Substanzen, insbesondere empfindlichen eine biologische Funktion aufweisenden Substanzen, wie Proteine, Rezeptoren, Liganden und Viren, wie für die Gentherapie geeigneten Viren, insbesondere AAV, eignet.

Die Erfindung wird durch die nachstehenden Beispiele erläutert.

### Beispiel 1: Herstellung eines erfindungsgemäßen Fᵥ-Antikörper-Konstruktes

Das scFᵥ-Antikörper-Konstrukt scFᵥ215 bindet an Drosophila RNA-Polymerase (Kontermann et al., (1995), Biol. Chem. 376, 473-481). Es wurde nach dem Verfahren von Breitling & Dübel, Methods in Molecular Medicine, Vol. 13 (1997) ed. U. Reischl, Humana press Inc., Totowa, N.Y., 581-591, in den Expressionsvektor pOPE101 (Genebank Nr.: Y14585) einkloniert. Dieser Expressionsvektor ermöglicht nach Zugabe von 50µM IPTG zu dem Kulturmedium der Bakterien eine Herstellung des scFᵥ-Antikörper-Konstruktes im Periplasma der Bakterien, aus dem er isoliert werden kann. Durch diese Klonierung wurden eine NcoI und eine HindIII-Restriktionsschnittstelle in die V_{H}-Region und eine MluI und eine NotI-Restriktionsschnittstelle an den Enden der jeweiligen V-Regionen eingeführt. Die für das scFᵥ215-Antikörper-Konstrukt kodierende DNA wurde dann in den E.coli Phagen-Display-Vektor pSEX81 (Genebank Nr.: Y14584) über die Restriktionsschnittstellen NcoI und NotI einkloniert, wodurch pSEX81-215(Yol) erhalten wurde. Dieser Expressionsvektor ermöglicht das Phagen-Display von Antikörper-Konstrukten, indem das scFᵥ-Antikörper-Konstrukt als aminoterminales Fusionsprotein mit dem Minor Coat Protein (pIII) eines filamentösen Phagen der M13-Gruppe exprimiert, und damit eine Oberflächenpräsentation des scFᵥ-Antikörper-Konstruktes erreicht wird. Die Sequenzidentität mit der geplanten Sequenz wurde duch DNA-Sequenzierung des Inserts überprüft.

Ferner wurde der Linker zwischen der V_{H}- und V_{L}-Region des scFᵥ-215-Antikörper-Konstruktes durch Peptide ersetzt, die für eine Vielzahl unterschiedlicher Sequenzen kodierten. Diese Peptide wurden in 10 verschiedenen Längen konstruiert, indem Peptid-Bibliotheken aus Oligonukleotiden hergestellt wurden, die anstelle der für den Linker zwischen der V_{H}- und V_{L}-Region aus pOPE101-215(Yol) kodierenden DNA in diesen Vektor eingesetzt wurden. Hierzu wurde im einzelnen wie folgt vorgegangen:

Es wurden 10 verschiedene Gemische (Wobbel-Bibliotheken) von Oligonukleotiden synthetisiert (LINKLIB6-LINKLIB15), die für 10 verschiedene Peptid-Längen kodierten (nachstehende Tab. 1). In diesen Oligonukleotiden wurden 6-15 Codons (NNB, wobei N alle Basen und B die Basen G, C, T bedeuten) randomisiert. Ferner wurden die randomisierten Bereiche jeweils am 5'-Ende des Oligonukleotides mit einer HindIII-Restriktionsschnittstelle und den 10 davorliegenden Nukleotiden der V_{H}-Region, wie sie aus der Sequenz des scFᵥ-Antikörper-Konstruktes hervorgehen, und am 3'-Ende mit einer MluI-Restriktionsschnittstelle und den darauf folgenden 10 Nukleotiden der V_{L}-Region, wie sie aus der Seqenz des scFᵥ-Antikörper-Konstruktes hervorgehen, versehen. Zwei weitere Oligonukleotid-Primer (RANFOR und RANBACK) wurden für die PCR-Amplifikation dieser 10 Oligonukleotid-Bibliotheken hergestellt, wobei die Sequenz des ersten Oligonukleotid-Primers der Sequenz des 5'-Endes des randomisierten Oligonukleotides entspricht, soweit die Sequenz noch keine Wobbel-Codons enthält, und die Sequenz des zweiten Oligonukleotid-Primers dem Gegenstrang des 3'-Endes des randomisierten Oligonukleotides entspricht, ab der Stelle, wo dieses keine Wobbel-Codons mehr enthält.

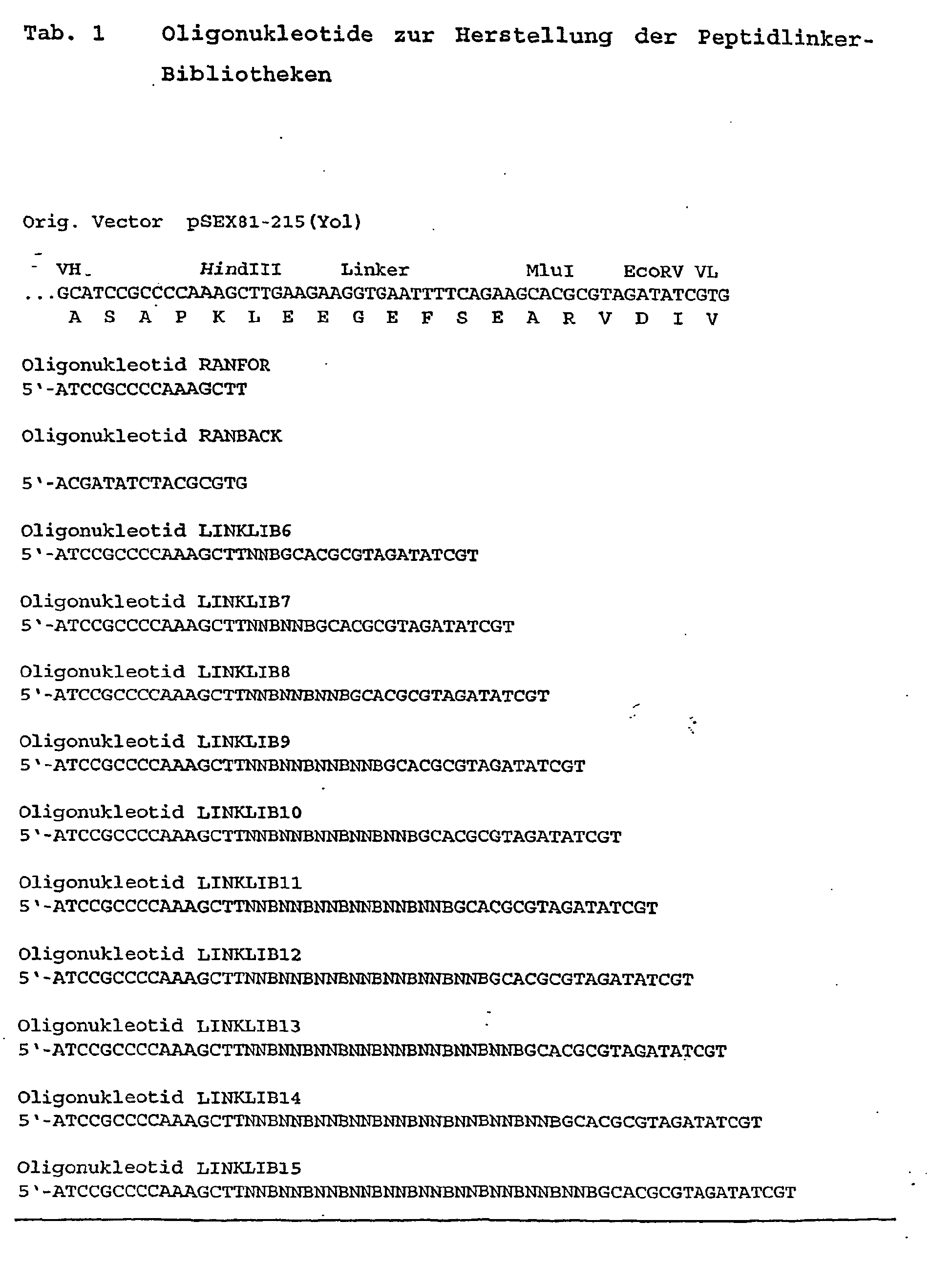

Mit RANFOR und RANBACK als Primer und den randomisierten 10 verschiedenen Gemischen (Wobbel-Bibliotheken) von Oligonukleotiden (LINKLIB6-LINKLIB15) als Template wurden 10 entsprechende PCR-Reaktionen mit jeweils 15 Zyklen durchgeführt, die zu einer Amplifikation der für die Peptide kodierenden DNA-Sequenzen führten. Die PCR-Produkte wurden über Agarosegelelektrophorese gereinigt. Nach Phenolisierung, Restriktionsverdau mit MluI und HindIII und erneuter gelelektrophoretischer Reinigung wurden die PCR-Produkte in den ebenfalls mit MluI und HindIII geschnittenen Expressionsvektor pSEX81-215(Vol) einkloniert. Dazu wurden die Ligationsprodukte mittels Elektroporation in jeweils 50µl kompetente E.coli X11blue-Zellen elektrotransformiert und auf Nährmedium-Agarplatten mit Ampicillin zur Selektion ausplattiert und über Nacht bei 37°C angezogen. Die erhaltenen Klone wurden dann in 500µl LB-Medium aufgenommen und amplifiziert und durch Zugabe von Helferphagen wurde die Bildung von Fᵥ-Antikörper-Konstrukte produzierenden Phagen induziert (Welschof et al., Proc. Natl.Acad.Sci.USA 94, (1997) 1902-1907). Die Phagen trugen jeweils die V_{H}- und V_{L}-Region des scFᵥ215-Antikörper-Konstruktes, wobei diese aber mit verschiedenen Peptide (10) verbunden waren. Jeweils 10¹³ Phagen dieser 10 verschiedenen Phagensuspensionen wurden in ein Polystyrolröhrchen gegeben, das vorher mit dem Antigen 215-βGal, einem aus E.coli-Inklusionskörpern gereinigten Fusionsprotein aus β-Galaktosidase und dem Epitop-Peptid des scFᵥ215-Antikörper-Konstruktes (Kontermann et a., (1995), supra) und danach mit 2 % Milchpulver (als Blocklösung) beschichtet worden war ("panning"). Nach einer Inkubation von 6 Stunden bei Raumptemperatur wurden die Phagensuspensionen abgesaugt und das Polystyrolröhrchen wurde 20 mal mit PBS gewaschen, um ungebundene Phagen zu entfernen. Danach erfolgte eine Elution der gebundenen Phagen mit steigenden Konzentrationen an Nickelchlorid in PBS (0,1, 0,5; 0,7; 1, 1,5; 2, 3; 4,5M). Die Eluate wurden zur Neutralisation des Nickels mit in PBS aufgeschlämmter Chelating Sepharose (Pharmacia) versetzt, und nach 15 min mit Plating-Bakterien (E. coli X11blue) gemischt. Nach einer Stunde Infektion bei 37°C wurden die Plating-Bakterien auf Ampicillin-Selektionsmedium ausplattiert. Die erhaltenen Klone wurden jeweils wieder zur Bildung von Fᵥ-Antikörper-Konstrukten produzierenden Phagen induziert. Drei weitere Runden des "Pannings" wurden, wie vorstehend beschrieben, durchgeführt, wobei diesmal aber nur mit der Konzentration eluiert wurde, bei der das Eluat der primären Elution erhalten wurde. Nach insgesamt 4 Panning-Runden waren bei 26 von 90 Ansätzen Kolonien zu finden, wobei bei diesen Elutionskonzentrationen über 0,5M NiCl vorlagen. Von den 26 Platten wurden 12 ausgewählt und von diesen jeweils 10 einzelne Klone gepickt und zur Bildung von Fᵥ-Antikörper-Konstrukte produzierenden Phagen induziert. Die erhaltenen 120 Einzelklone wurden in einem Phagen-ELISA in 96-well-ELISA-Platten untersucht, wobei als Antigen 215-βGal verwendet und der Nachweis mit dem monoklonalen Antikörper B62-FE2 (Michael, B. et al., (1994) J. Imm. Methods 171, 103-109) geführt wurde. Als Kontrolle dienten der Originalvektor pSEX81-215(Yol) und für jeden Klon wurden zwei wells eingesetzt, eines ohne und eines mit 1M NiCl während der Inkubation der Phagen mit dem Antigen. Von den 120 Einzelklonen ergaben 63 eine positive Reaktion (=Antigenbindung), von diesen waren 45 mit Nickel kompetierbar. Die 45 kompetierbaren Klone wurden gepoolt und erneut als Phagen verpackt, die dann wiederum einer "panning"-Runde unterworden wurden, bei der die Elution mit 0,5M NiCl in PBS erfolgte. Von den eluierten Phagen wurden nach Reinfektion und Amplifikation in plating-Bakterien 12 Kolonien gepickt und ihr Peptidlinker zwischen der V_{H}- und V_{L}-Region auf DNA-Ebene sequenziert. Alle 12 Phagen wiesen eine einheitliche DNA-Sequenz auf, die für den Peptidlinker HPHHHHE kodiert.

### Beispiel 2: Verwendung des erfindungsgemäßen Fᵥ-Antikörper-Konstruktes zur Anreicherung und Reinigung einer Substanz

Nach Umklonierung eines das Peptid HPHHHHE aufweisenden scFᵥ215-Antikörper-Konstruktes nach Beispiel 1 in pOPE51 wurde das scFᵥ-Antikörper-Konstrukt in E. coli produziert, gereinigt und über das ungepaarte Cystein nahe des Carboxyterminus kovalent an eine Affinitätschromatographiesäule gekoppelt. Über diese Säule wurde ein gereinigtes, aktives Fusionsprotein aus dem M13-Phagenprotein pIII, bei dem die letzten 24 Aminosäurereste deletiert und durch das Epitop-Peptid des scFᵥ215-Antikörper-Konstruktes ersetzt wurden, gegeben. Die anschließende Elution erfolgte mit einem Gradient von 0 - 1M NiCl in PBS. Das Fusionsprotein wurde ab einer Konzentration von 0,22M eluiert, mit dem Peak bei 0,4M. Die Ausbeute betrug 72%. Es erfolgte eine Dialyse in PBS zur Entfernung des Nickelchlorids. Die spezifische Aktivität des pIII (gemessen im Infektionsinhibitionstest auf F-Pili von E. coli) entsprach jener eines als Kontrolle eingesetzten, nicht über die Affinitätschromatographiesäule gegebenen pIII. Diese Ergebnisse wurden mehrfach in Versuchen bestätigt, in denen die gleiche Affinitätschromatographiesäule verwendet wurde.

Somit zeigte es sich, daß bei einem erfindungsgemäßen Fᵥ-Konstrukt die Affinität zu einer zu bindenden Substanz beeinflußt werden kann, d.h. die Substanz kann z.B. wieder abgelöst werden, ohne daß eine wesentliche Beeinträchtigung des Fᵥ-Konstruktes, z.B. in seiner Wiederverwendbarkeit, und der Substanz, z.B. in ihrer Aktivität, erfolgt ist.

## Patentansprüche

1. Fᵥ-Konstrukt mit beeinflußbarer Affinität zu einer zu bindenden Substanz, wobei die variablen Regionen des Fv-Konstrukts über Peptide miteinander verbunden sind, die Peptide Bindungsstellen für Effektormoleküle enthalten und die Bindung von Effektormolekülen an die Bindungsstellen der Peptide zu einer allosterischen Modulation des Fv-Konstrukts führt, die die Affinität des Fv-Konstrukts zur bindenden Substanz verstärkt oder reduziert.

2. Fᵥ-Konstrukt nach Anspruch 1, wobei das Fᵥ-Konstrukt ein einzelkettiges Fᵥ-Konstrukt ist.

3. Fᵥ-Konstrukt nach Anspruch 1 oder 2, wobei das Fᵥ-Konstrukt ein Fᵥ-Antikörper-Konstrukt ist.

4. Fᵥ-Konstrukt nach Anspruch 1 oder 2, wobei das Fᵥ-Konstrukt ein Fᵥ-T Zell-Rezeptor-Konstrukt ist.

5. Fᵥ-Konstrukt nach einem der Ansprüche 1 bis 4, wobei das Fᵥ-Konstrukt monospezifisch ist.

6. Fᵥ-Konstrukt nach einem der Ansprüche 1 bis 4, wobei das Fᵥ-Konstrukt bispezifisch ist.

7. Fᵥ-Konstrukt nach einem der Ansprüche 1 bis 6, wobei die Effektormoleküle Ionen sind.

8. Fᵥ-Konstrukt nach Anspruch 7, wobei die Ionen bivalente oder trivalente Ionen sind.

9. Fᵥ-Konstrukt nach Anspruch 7 oder 8, wobei die Ionen Metallionen sind.

10. Fᵥ-Konstrukt nach Anspruch 9, wobei die Metallionen Nickel-, Kupfer- oder Zinkionen sind.

11. Fᵥ-Konstrukt nach Anspruch 9 oder 10, wobei die Bindungsstellen für die Metallionen Zinkfinger oder HIS-Repeats sind.

12. Fᵥ-Konstrukt nach einem der Ansprüche 1 bis 6, wobei die Effektormoleküle Antikörper sind.

13. Fᵥ-Konstrukt nach einem der Ansprüche 1 bis 12, wobei das Fᵥ-Konstrukt in Verbindung mit einer Matrix vorliegt.

14. Fᵥ-Konstrukt nach Anspruch 13, wobei die Matrix eine Chromatographiesäule ist.

15. Fᵥ-Konstrukt nach einem der Ansprüche 1 bis 14, wobei die zu bindende Substanz ein Protein, ein Rezeptor, ein Ligand, ein Virus oder eine synthetische Verbindung ist.

16. Verfahren zur Herstellung des Fᵥ-Konstruktes nach einem der Ansprüche 2 bis 12, bei dem eine DNA, die für variable Regionen kodiert, wobei die Regionen über Bindungsstellen für Effektormoleküle aufweisende Peptide miteinander verbunden sind, in einem Expressionsvektor inseriert und über diesen in Zellen exprimiert wird.

17. Verwendung des Fᵥ-Konstruktes nach einem der Ansprüche 1 bis 15 zur Anreicherung und Reinigung von Substanzen.

18. Verwendung nach Anspruch 17, wobei die Anreicherung und Reinigung im Rahmen einer Affinitätschromatographie erfolgt.

## Claims

1. An Fᵥ construct having an influenceable affinity for a substance to be bound, the variable regions of the Fᵥ construct being linked via peptides, the peptides containing binding sites for effector molecules and the binding of effector molecules to the binding sites of the peptides resulting in an allosteric modulation of the Fᵥ construct which enhances or reduces the affinity of the Fᵥ construct for the binding substance.

2. The Fᵥ construct according to claim 1, wherein the Fᵥ construct is a single-chain Fᵥ construct.

3. The Fᵥ construct according to claim 1 or 2, wherein the Fᵥ construct is an Fᵥ antibody construct.

4. The Fᵥ construct according to claim 1 or 2, wherein the Fᵥ construct is an Fᵥ T-cell receptor construct.

5. The Fᵥ construct according to any of claims 1 to 4, wherein the Fᵥ construct is monospecific.

6. The Fᵥ construct according to any of claims 1 to 4, wherein the Fᵥ construct is bispecific.

7. The Fᵥ construct according to any of claims 1 to 6, wherein the effector molecules are ions.

8. The Fᵥ construct according to claim 7, wherein the ions are bivalent or trivalent ions.

9. The Fᵥ construct according to claim 7 or 8, wherein the ions are metal ions.

10. The Fᵥ construct according to claim 9, wherein the metal ions are nickel, copper or zinc ions.

11. The Fᵥ construct according to claim 9 or 10, wherein the binding sites for the metal ions are zinc fingers or HIS repeats.

12. The Fᵥ construct according to any of claims 1 to 6, wherein the effector molecules are antibodies.

13. The Fᵥ constructs according to any of claims 1 to 12, wherein the Fᵥ construct is available in connection with a matrix.

14. The Fᵥ construct according to claim 13, wherein the matrix is a chromatographic column.

15. The Fᵥ construct according to any of claims 1 to 14, wherein the substance to be bound is a protein, a receptor, a ligand, a virus or a synthetic compound.

16. A method of producing the Fᵥ construct according to any of claims 2 to 12, wherein a DNA coding for variable regions, the regions being linked via binding sites for peptides having effector molecules, is inserted in an expression vector and expressed in cells via the latter.

17. Use of the Fᵥ construct according to any of claims 1 to 15 for enriching and purifying substances.

18. Use according to claim 17, wherein the enrichment and purification is carried out on the basis of an affinity chromatography.

## Revendications

1. Elément de construction Fv possédant une affinité influençable pour une substance apte à se lier, dans laquelle des régions variables de l'élément de construction Fv sont reliées entre elles par des peptides, les peptides renfermant des sites de liaison pour les molécules d'effecteurs et la liaison des molécules d'effecteurs aux sites de liaison des peptides conduit à une modulation allostérique de l'élément de construction Fv, qui amplifie ou réduit l'affinité de l'élément de construction Fv aux substances à lier.

2. Elément de construction Fv selon la revendication 1, **caractérisé en ce qu'**il est un élément de construction Fv à chaîne unique.

3. Eléments de construction Fv selon la revendication 1 ou 2, **caractérisé en ce que** l'élément de construction Fv est un élément de construction d'anticorps Fv.

4. Elément de construction Fv selon la revendication 1 ou 2, **caractérisée en ce qu'**il est un élément de construction récepteur de cellules Fv-T.

5. Elément de construction Fv selon l'une des revendications 1 à 4, **caractérisé en ce qu'**il est mono-spécifique.

6. Elément de construction Fv selon l'une des revendications 1 à 4, **caractérisé en ce qu'**il est bi-spécifique.

7. Elément de construction Fv selon l'une des revendications 1 à 6, **caractérisé en ce que** les molécules d'effecteurs sont des ions.

8. Elément de construction Fv selon la revendication 7, **caractérisé en ce que** les ions sont des ions bivalents ou trivalents.

9. Elément de construction Fv selon la revendication 7 ou 8, **caractérisé en ce que** les ions sont des ions métalliques.

10. Elément de construction Fv selon la revendication 9, **caractérisé en ce que** les ions métalliques sont représentés par les ions nickel, cuivre ou zinc.

11. Elément de construction Fv selon la revendication 9 ou 10, **caractérisé en ce que** les sites de liaison des ions métalliques sont des sites de zinc ou des éléments répétitifs HIS.

12. Elément de construction Fv selon l'une des revendications 1 à 11, **caractérisé en ce que** les molécules d'effecteurs sont des anticorps.

13. Elément de construction Fv selon l'une des revendications 1 à 12, **caractérisé en ce qu'**il est en liaison avec une matrice.

14. Elément de construction Fv selon la revendication 13, **caractérisé en ce que** la matrice est une colonne de chromatographie.

15. Elément de construction Fv selon l'une des revendications 1 à 14, **caractérisé en ce que** la substance de liaison est une protéine, un récepteur, un ligand, un virus ou une liaison synthétique.

16. Procédé de préparation d'éléments de construction Fv selon l'une des revendications 2 à 12, dans lequel un ADN, codant les régions variables, où les peptides possédant des régions pour les molécules d'effecteurs au niveau des sites de liaison, est inséré dans un vecteur d'expression et exprimé par ceux-ci en cellules.

17. Utilisation de l'élément de construction Fv selon l'une des revendications 1 à 15 pour l'enrichissement et le nettoyage des substances.

18. Utilisation selon la revendication 17, **caractérisée en ce que** l'enrichissement et le nettoyage sont réalisés dans le cadre d'une chromatographie d'affinité.
